# EUROPEAN PATENT SPECIFICATION

(11) **EP 4 063 834 B1**
(45) Date of publication and mention of the grant of the patent: **05.11.2025**
(21) Application number: 19953561.8
(22) Date of filing: 22.11.2019
(51) Int. Cl.: G01N 21/78, G01N 33/50, G01N 33/04

(54) **DAIRY SOLUTION ANALYSIS METHOD AND PORTABLE DAIRY SOLUTION ANALYSIS DEVICE**
VERFAHREN ZUR ANALYSE VON MILCHLÖSUNGEN UND TRAGBARE VORRICHTUNG ZUR ANALYSE VON MILCHLÖSUNGEN
PROCÉDÉ D'ANALYSE DE SOLUTION LAITIÈRE ET DISPOSITIF D'ANALYSE DE SOLUTION LAITIÈRE PORTABLE

(43) Date of publication of application: 28.09.2022
(73) Proprietor: BOE Technology Group Co., Ltd., Beijing 100015 (CN)
(72) Inventor: QIN, Qin, Beijing 100176 (CN); ZHANG, Yufan, Beijing 100176 (CN); LI, Jing, Beijing 100176 (CN); AN, Guangming, Beijing 100176 (CN); FENG, Mengli, Beijing 100176 (CN)
(74) Representative: Brötz, Helmut
(86) International application number: PCT/CN2019/120332
(87) International publication number: WO 2021/097816

(56) References cited:
- EP-A1- 3 847 457
- WO-A1-2014/051425
- WO-A1-2017/100918
- WO-A1-2019/171368
- CN-A- 101 014 856
- CN-A- 102 778 546
- CN-A- 105 866 416
- CN-A- 108 387 574
- CN-A- 109 142 256
- US-A1- 2007 289 536
- US-A1- 2009 298 191
- US-A1- 2016 082 165
- US-A1- 2016 139 156
- US-A1- 2019 088 357

## Description

### TECHNICAL FIELD

The present disclosure relates to a dairy solution analysis method and an electronic device thereof and a portable dairy solution analysis device.

### BACKGROUND

Infants, the elderly, and patients usually supplement their body needs with dairy solutions. Dairy solutions may be breast milk, cow's milk, goat's milk, formula milk, and the like. However, incorrect consumption of dairy solutions may not only fail to fulfill the purpose of replenishing needed nutrients for body, but may also have adverse effects on health. Therefore, there is a need to analyze compositions of dairy solutions.

At present, devices that can be used for dairy solution analysis are mainly concentrated in medium to large-sized hospitals, and all of them are large-scale testing devices. Two technologies are usually used in large-scale testing devices for dairy solution analysis: infrared spectrum analysis technology and/or ultrasonic detection technology. Large-scale devices for dairy solution analysis using these two technologies are bulky, so only large diagnostic centers such as class-A hospitals are equipped with such devices. At the same time, these large-scale testing devices require trained professionals to operate. It is very time-consuming and laborious for users to visit hospitals to test compositions of dairy solutions. US 2016/139156 A1 discloses methods, systems and apparatuses for monitoring a physiological condition of a user. WO 2014/051425 A1 discloses a test strip reading device and a method for reading a test strip. WO 2019/171368 A1 discloses a system, a device and a method for identifying and monitoring breast milk composition. US 2016/082165 A1 discloses systems, methods and devices for milk expression. WO 2017/100918 A1 discloses a portable liquid analyzing device. US 2009/298191 A1 discloses lateral flow and flow-through bioassay devices. EP 3 847 457 A1 discloses devices, test strip and methods for testing breastmilk and providing feedback for improving the quality thereof.

### SUMMARY

At least one embodiment of the present disclosure provides a dairy solution analysis method, comprising: controlling a plurality of light emitting diodes to emit light of different wavelengths according to a type of a substance to be tested, wherein different light emitting diodes emit light with different wavelengths; detecting, by internal sensors, a test value of at least one indicator of a dairy solution by emitting lighting on different wave lengths of light on a plurality of test papers, based on a colorimetric biochemical testing method; wherein the detecting including: the internal sensors absorb light reflected by the test papers and determining the test value of the at least one of indicators by a degree of light transmission of the test papers; calibrating the test value of the at least one indicator of the dairy solution according to the calibration information; outputting an analysis result of the dairy solution based on a calibrated test value of the at least one indicator of the dairy solution, wherein the plurality of test papers are located in a test paper housing component, the test paper housing component is placed on a test paper placement platform, wherein in the case where the calibration information includes the adjustment factor of the test paper, an actual value of the at least one indicator of the dairy solution is obtained by applying the adjustment factor of the test paper to the test value of the at least one indicator of the dairy solution

At least one embodiment of the present disclosure provides a portable dairy solution analysis device, comprising: a processor configured to control a plurality of light emitting diodes to emit light of different wavelengths according to a type of a substance to be tested, wherein different light emitting diodes emit light with different wavelengths; a detector configured to detect a test value of at least one indicator of a dairy solution by emitting lighting on different wavelengths of light on a plurality of test papers, based on a colorimetric biochemical testing method; wherein the detecting including: the internal sensors absorb light reflected by the test papers and determining the test value of the at least one of indicators by a degree of light transmission of the test papers; the processor configured to obtain calibration information, wherein the calibration information includes at least one of: a production batch of the test paper, an adjustment factor of the test paper, a manufacturer of the test paper, a production time of the test paper, and a production condition of the test paper and calibrate the test value of the at least one indicator of the dairy solution according to the calibration information; the processor configured to output an analysis result of the dairy solution, based on a calibrated test value of the at least one indicator, wherein the plurality of test papers are located in a test paper housing component, the test paper housing component is placed on a test paper placement platform, wherein in the case where the calibration information includes the adjustment factor of the test paper, an actual value of an indicator is obtained by applying the adjustment factor of the test paper to the test value.

At least one embodiment of the present disclosure provides a dairy solution analysis system, comprising: the above portable dairy solution analysis device; an electronic device configured to output the analysis result of the dairy solution.

At least one embodiment of the present disclosure provides a dairy solution analysis electronic device, comprising: a processor; a memory having computer instructions stored thereon, which when executed by the processor, the dairy solution analysis method is implemented.

### BRIEF DESCRIPTION OF THE DRAWINGS

In order to illustrate the technical solution of embodiments of the present disclosure more clearly, accompanying drawings of the embodiments will be briefly introduced below. Obviously, the accompanying drawings described below involve only some of the embodiments of the present disclosure and are not intended to limit the present disclosure.

In order to illustrate the technical solution of at least one embodiment of the present disclosure more clearly, drawings required for description of the embodiment will be briefly introduced below. The drawings in the following description are merely exemplary embodiments of the present disclosure.
Fig. 1A is a flowchart illustrating a dairy solution analysis method according to at least one embodiment of the present disclosure.
Fig. 1B is a diagram illustrating a dairy solution analysis device according to at least one embodiment of the present disclosure.
Fig. 1C is a structural diagram illustrating a portable dairy solution analysis device according to at least one embodiment of the present disclosure.
Fig. 2 is another schematic diagram illustrating the portable dairy solution analysis device according to at least one embodiment of the present disclosure.
Fig. 3A is another schematic diagram illustrating the portable dairy solution analysis device according to at least one embodiment of the present disclosure.
Fig. 3B is another flowchart illustrating a dairy solution analysis method according to at least one embodiment of the present disclosure.
Fig. 4 is yet another schematic diagram illustrating the portable dairy solution analysis device according to at least one embodiment of the present disclosure.
Fig. 5 is schematic a diagram illustrating a dairy solution analysis system according to at least one embodiment of the present disclosure.
Fig. 6 is a structural diagram illustrating a dairy solution analysis electronic device according to at least one embodiment of the present disclosure.

### DETAILED DESCRIPTION

In order to make objects, technical solutions and advantages of the embodiments of the present disclosure more obvious, the technical solutions of the embodiments of the present disclosure will be described below clearly and thoroughly in connection with the accompanying drawings. Apparently, the described embodiments are only a part but not all of the embodiments of the present disclosure. Based on the described embodiments of the present disclosure, all other embodiments obtained by a person of ordinary skill in the art without inventive labor fall within the scope of the present disclosure.

Unless otherwise specified, all the technical and scientific terms used herein have the same meanings as commonly understood by one of ordinary skill in the art to which the present disclosure belongs. The terms "first," "second," and the like as used in the present disclosure are not intended to indicate any sequence, amount or importance, but are used to distinguish various components. Also, the terms such as "a," "an," "the," and the like are not intended to limit the amount, but indicate that there is at least one. The terms "comprise," "include," and the like are intended to specify that elements or objects stated before these terms encompass elements or objects and equivalents thereof listed after these terms. For ease of description, in some of the drawings, "up," "down," and the like are given to indicate relative position relationship only, and when an absolute position of the described objects changes, the relative position relationship may change accordingly.

Nowadays, testing devices that can be found on the market for testing substance content of various liquids, such as breast milk, cow's milk, formula, etc., are mainly large-scale testing devices. These testing devices are expensive and complex to operate, and also require special training for operators. The above-mentioned testing devices are mainly concentrated in hospitals or testing institutions, to provide substance content test for those in need. As a result, testing of some liquids, such as breast milk, needs to be performed in hospitals or specialized testing institutions, which is time-consuming and laborious. Therefore, it is difficult for those in need to perform frequent testing. For liquids that require frequent testing and regular monitoring of their substance content, such as, breast milk, testing of their substance content is greatly limited. Therefore, it is important to provide an analyzer that is portable, compact, easy to operate, and can be used at home to test substance content of liquids such as breast milk.

Lab-on-a-chip is a technique in which basic units of operation such as sample preparation, biological and chemical reactions, separation and testing, and the like, are integrated or substantially integrated into a chip of, for example, a few square centimeters, so as to perform different biological or chemical processes and to analyze their products. Signals generated within the chip need to be detected. The most commonly used detection methods include laser-induced fluorescence, mass spectrometry, ultraviolet, chemiluminescence and the like.

A portable dairy solution analysis device, system, and methods thereof provided by one or more embodiments of the present disclosure will be described in detail below in connection with the accompanying figures.

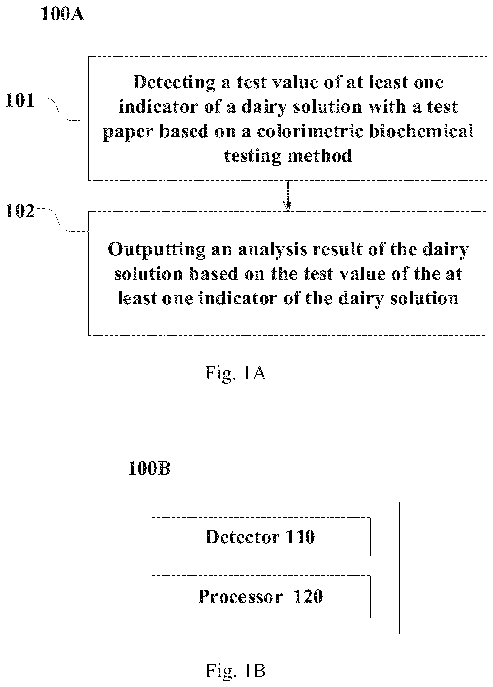

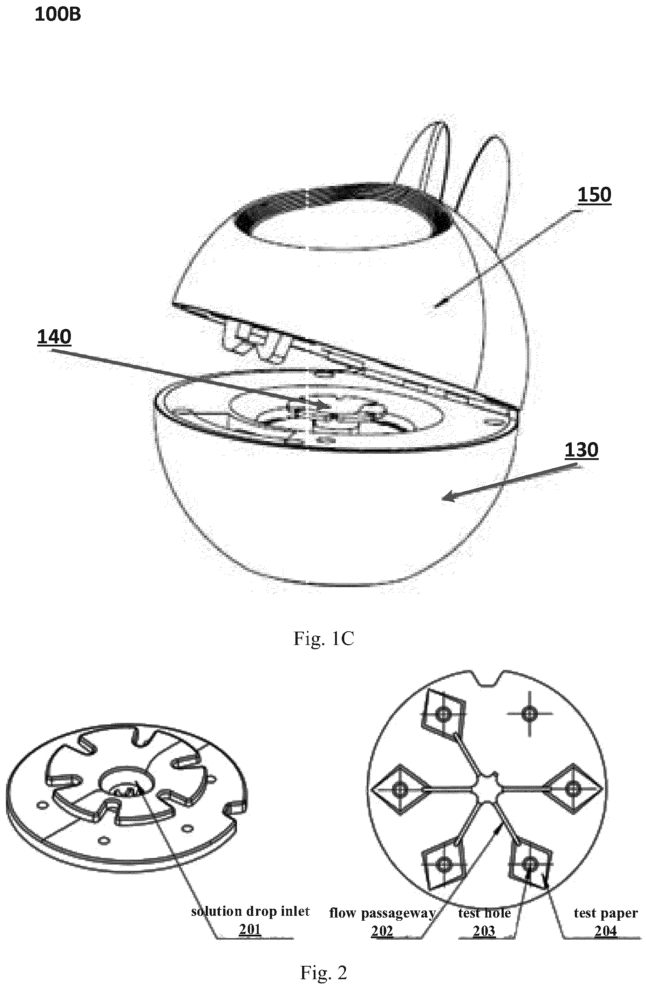

Fig. 1A is a flowchart illustrating a dairy solution analysis method 100A according to at least one embodiment of the present disclosure. Fig. 1B is a structural diagram illustrating a dairy solution analysis device 100B according to at least one embodiment of the present disclosure. Fig. 1C is a schematic diagram illustrating a portable dairy solution analysis device 100B according to at least one embodiment of the present disclosure.

Referring to Fig. 1A, the dairy solution analysis method 100A includes operation 101 and operation 102. The dairy solution analysis method 100A may be performed by the portable dairy solution analysis device 100B illustrated in Figs. 1B and 1C, which may also be performed by any other electronic devices. Referring to Figs. 1B and 1C, the portable dairy solution analysis device 100B may include a detector 110 and a processor 120. As an example, the portable dairy solution analysis device 100B may be less than 40 centimeters in length, width, and height to allow for portable use in multiple scenarios such as home, travel, lactation rooms, maternity centers, health care facilities, and the like.

In operation 101, a test paper is used to detect a test value of at least one indicator of a dairy solution based on a colorimetric biochemical testing method. Optionally, the dairy solution may be, for example, breast milk, cow's milk, goat's milk, formula milk and the like. Optionally, the detector 110 may be configured to perform the operation 101. For example, the colorimetric biochemical testing method may utilize a color change in the test paper to determine the test result. Depending on different test papers, the colorimetric biochemical testing method may be used for testing of different indicators, for example, conventional physical and chemical indicators, biochemical indicators, and the like. The colorimetric biochemical testing method described in the present disclosure includes, but is not limited to, dry chemistry, immunochromatography (based on colored latex particles), chemiluminescence, and the like. The tester 110 may determine and derive a plurality of indicators based on the color change of the test paper upon action with the dairy solution.

For example, the detector 110 may include one or more of an upper component 150, a lower component 130, a light-emitting element, a photoelectric sensor, and a test paper placement platform 140. The test paper is placed in any test paper housing component (e.g., a test chip) and then placed on the test paper placement platform. The lower component 130 may carry the test chip and optically inspect the test paper in the test chip. The test chip is placed on the test paper placement platform 140 in the lower component 130. The test paper placed on the test paper placement platform is illuminated by light emitted by the light-emitting element. The light reflected from the test paper is detected by the photoelectric sensor, and the color of the test paper is inspected accordingly to determine various indicators.

The light-emitting element may be included within the lower component 130 or the upper component 150. The light-emitting element may be controlled to radiate its emission light onto the test paper soaked with the dairy solution. Since a single test chip includes a plurality of test papers, and these test papers may react independently with corresponding components of the dairy solution, different test papers may display different colors. Shades of the colors on the test papers are proportional to concentrations of respective components in the dairy solution. The light-emitting element is a Light Emitting Diode (LED), such as a silicon photodiode and the like. A light-emitting diode may emit light of a specific wavelength (e.g., infrared light, red light, green light, etc.). A light-emitting diode of a specific wavelength may be selected according to a type of a substance to be tested. There are a plurality of light-emitting diodes, and different light-emitting diodes emit light of different wavelengths, thereby achieving testing for a variety of substances. For example, the light-emitting element may be selected to emit light with a wavelength of 630 nm, to test the content of lactose and fat in a liquid to be tested. The light-emitting element may also be selected to emit light with a wavelength of 660 nm, to test the content of calcium and protein in a liquid to be tested.

The detector 110 determines the respective indicators by testing the color of the test paper. The detector 110 includes internal sensors (e.g., photoelectric sensors). These internal sensors may be located within the lower component 130 or the upper component 150. The internal sensors absorb light reflected by the test paper and achieve testing of the plurality of indicators by a degree of light transmission of the test paper. For example, the internal sensors test reflection of lights by the test paper soaked with a certain amount of dairy solution, and convert the light into a digital signal. One or more analysis modules/processors may also be included in the detector 110. These analysis modules may analyze the digital signal to determine the indicators (e.g., substance contents/concentrations) of the dairy solution.

Optionally, the plurality of indicators of the dairy solution described above include one or more of: fat content, protein content, lactose content, calories, zinc content, and calcium content. A calorie is a unit of heat, which is the amount of heat required to raise the temperature of one gram of water by one degree Celsius (°C). One calorie is equal to 4.1868 joules. Calories may be calculated from indicators such as fat content, protein content, lactose content, etc. For example, 1g of lactose/protein yields approximately 4,000 calories, and 1g of fat yields approximately 9,000 calories. Therefore, the higher the fat content, the higher the calorie content may be estimated.

Those skilled in the art should appreciated that the dairy solution analysis method 100A according to at least one embodiment of the present disclosure may also be used to detect test values of other indicators associated with the dairy solution, as long as the indicators can be tested by the colorimetric biochemical testing method.

In operation 102, an analysis result of the dairy solution is output based on the test value of the at least one indicator of the dairy solution. The processor 120 may be configured to perform the operation 102.

Optionally, the processor 120 may be included in the upper component 150 or the lower component 130. When the upper component 150 is open, a user may place the test paper housing component (e.g., a test chip) containing the test paper on the test paper placement platform 140. And when the upper component 150 is closed, testing of the dairy solution may be started in a shaded environment. By opening and closing the upper component 150, it is possible to control the amount of light transmitted/reflected from the test paper and reduce an influence of external ambient light. For example, when the upper assembly 150 is closed, ambient light will not be able to enter within the portable dairy solution analysis device 100Band thus cannot affect internal sensors' detection of light reflection/transmission on the test paper.

Optionally, the analysis result of the dairy solution includes whether the test value of the at least one indicator of the dairy solution deviates from a reference range of normal values. The reference range of normal values may be determined based on age in month or gender of an infant. When feeding an infant, nutritional requirements for the infant will vary according to the increasing age of the infant and the gender of the infant. For example, within the first seven days of the infant's birth, the infant will require a large amount of sodium, potassium, chlorine, protein, vitamins, minerals, antibodies and so on. In this case, the portable dairy solution analysis device 100B may set reference ranges for normal values of relevant indicators to be relatively high, to ensure the infant's nutrition. As the infant grows, the infant may need to be supplemented with more fat, water, sugar, and other substances to meet daily metabolic requirements. In this case, the portable dairy solution analysis device 100B may set reference ranges for normal values of sodium, potassium, chlorine and the like to be relatively low, and reference ranges for normal values of fat content, lactose content, and energy to be relatively high.

The portable dairy solution analysis device 100B may also include a display screen or speaker. In the case that the test value of the at least one indicator of the dairy solution is within a reference range for normal values, the processor 120 determines that the test value of this indicator of the tested dairy solution is normal. The analysis result that the test value of this indicator is normal may then be output to the display screen or speaker by the processor 120. The analysis result may be displayed on the display screen or played by the speaker. In the case that the test value of the at least one indicator of the dairy solution deviates from a reference range for normal values, the processor 120 may determine that the test value of the indicator of the tested dairy solution is abnormal. The analysis result that the test value for the indicator is abnormal may then be output to the display screen or speaker by the processor 120. For example, the display screen may indicate whether indicators are normal or abnormal by displaying value of the indicators in different colors (e.g., normal indicators are displayed in green, while abnormal indicators are displayed in red). Specific numeric values of various indicators described above and corresponding reference ranges for normal values of these indicators may also be displayed on the display screen. If an indicator is abnormal, the speaker may also remind the user that the indicator of the dairy solution is abnormal by means of alarm sounds/voice prompts, etc. The present disclosure does not further limit the presentation of analysis results of the processor 120 of the portable dairy solution analysis device 100B, as long as it can indicate normal/abnormal values of at least one of indicators of the tested dairy solution.

The portable dairy solution analysis device 100B according to at least one embodiment of the present disclosure is convenient, compact, and easy to operate. A user can analyze and test a plurality of indicators of a dairy solution without professional training. The portable dairy solution analysis device 100B can be used in multiple scenarios such as home, travel, lactation rooms, maternity centers, health care facilities, and the like, to meet a user's need to analyze dairy solution components anytime and anywhere.

With the dairy solution analysis method 100A and/or the portable dairy solution analysis device 100B, a user may learn whether indicators for various components of a dairy solution are met in real-time. For a user who is a lactating mother, when she wishes to control her diet to shape her body, or when she is unable to consume certain nutrients for various reasons, she may use the dairy solution analysis method 100A and/or the portable dairy solution analysis device 100B to monitor the quality of her own breast milk, so as to prevent a decreased quality of her breast milk due to improper diet. A lactating mother may also seek information about whether composition of frozen breast milk has changed, whether there is a change in the second child's breast milk compared with the past, whether the mother's poor physical condition may affect the quality of her breast milk, whether the energy of her breast milk is sufficient for the infant's growth, whether complementary foods should be added, and whether the infant needs to be weaned, among other things. Such mothers may obtain information on quality of their breast milk in real-time with the dairy solution analysis method 100A and/or the portable dairy solution analysis device 100B, so as to adjust their recipes and the way they feed their infants in time.

Fig. 2 is another schematic diagram illustrating the portable dairy solution analysis device 100B according to at least one embodiment of the present disclosure, which shows an example of a test paper housing component 200. The test paper housing component 200 is shown in the form of a test chip. Those skilled in the art should appreciate that the test paper housing component 200 may have other structures as well.

Referring to Fig. 2, the test paper housing component 200 includes a solution drop inlet 201, a flow passageway 202, a test hole 203, and a test paper 204. Optionally, a certain amount (e.g., about 0.1 ml) of dairy solution may be dropped onto the test paper housing member 200 through the drop inlet 201. The test paper housing member 200 may have the specific flow passageway 202 to distribute an equal amount of dairy solution into a plurality of test holes 203. The test paper 204 may be located below the test holes and soaked with the dairy solution. The test paper 204 may also be dried after being soaked with the dairy solution, and then tested after being dried. Test papers in various test holes may be the same or different, and different test papers may correspond to test for different indicators.

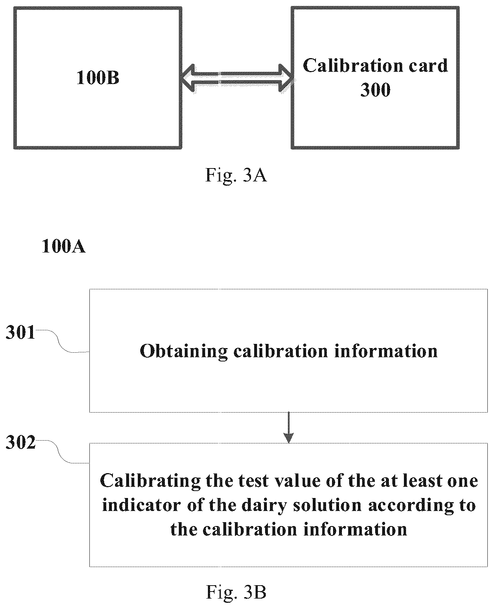

Fig. 3A is another schematic diagram illustrating the portable dairy solution analysis device 100B according to at least one embodiment of the present disclosure, which shows the portable dairy solution analysis device 100B interacting with a calibration card 300. Fig. 3B is another flowchart illustrating a dairy solution analysis method 100A according to at least one embodiment of the present disclosure.

Since test papers may have different production conditions during production, there may be slight differences in colors appearing by different batches of test papers soaked with a same dairy solution, which will affect measurement of relevant indicators. To this end, the dairy solution analysis method 100A may further include operation 301 and operation 302.

In operation 301, calibration information is obtained. In operation 302, based on the calibration information, the test value of the at least one indicator of the dairy solution is calibrated. The processor 120 may be configured to perform operation 301 and operation 302.

The calibration information includes any one or more of: a production batch, an adjustment factor, a manufacturer, a production time, a production condition of the test paper and the like. The calibration information may be located on the calibration card 300. The calibration card 300 may have a similar structure as the test chip described above, or may have a different structure than the test chip described above. For example, the calibration card 300 may also be a paper card with calibration information written on it. For example, the calibration card 300 may be attached directly to each test chip or may be a part of the test chip. The calibration card 300 may also be embedded in the test chip as a part of the test paper. The calibration card 300 may also be placed on the test paper placement platform 140, or inserted into other slots on the portable dairy solution analysis device 100B, or placed outside of any portable dairy solution analysis device 100B. It should be appreciated by those skilled in the art that the calibration card 300 may have any tangible or intangible structure, so long as it can perform the function of storing calibration information, which is not limited by the embodiments of the present disclosure. For example, the calibration card 300 may store information in the form of a two-dimensional code, a barcode, a specific value, and the like. The processor 120 may read information on the calibration card 300 by scanning the calibration card 300.

In the case where the calibration information includes the adjustment factor of the test paper, an actual value of an indicator may be obtained by applying the adjustment factor of the test paper to a test value. A function between actual values and test values of an indicator may be referred to as a calibration function. For example, assume that a value of a certain indicator tested by a certain batch of test papers is K. The actual value of the indicator should be K', where K' = a*K + b. The adjustment factor of the test paper may include a and b. The process of adjusting K to K' is the process of calibrating values of a plurality of indicators of the dairy solution. Those skilled in the art should appreciate that the above calibration manner using linear function is only an example, and that the relationship between actual values of an indicator and test values of the indicator is not limited to the linear function described above. The calibration function may be built into a memory of the portable dairy solution analysis device 100B to be read by the processor 120. The calibration function may also be stored on the calibration card 300 or on a test paper as a two-dimensional code, barcode, etc., to be read by the processor 120 along with other information such as the adjustment factor.

In the case where the calibration information includes one or more of information such as a production batch of the test paper, a manufacturer of the test paper, a production time of the test paper, a production condition of the test paper and the like, the processor 120 may also calibrate a test value of an indicator based on this information. For example, a two-dimensional code or barcode on the calibration card or test paper may be scanned by the processor 120, wherein the two-dimensional code or barcode stores the calibration information. Then a message containing letters and numbers may be read out from the two-dimensional code or barcode by the processor 120. The message may have a fixed format, for example, the first 8 digits indicate the production batch of the test paper, the middle 8 digits indicate the adjustment factor of the test paper, and the like. The present disclosure does not further limit how the calibration information may be stored in a two-dimensional code or barcode and the format of the message. Certainly, the calibration information may also be written on the calibration card 300 or on the test paper directly in text form. For example, the calibration card may be directly written with "test paper production batch: XXXX; manufacturer: AAAA," and the like. The calibration information on the calibration card 300 or the test paper is recognized by the processor 120 by using image recognition technologies. For example, the portable dairy solution analysis device 100B may include a camera that can capture information on the calibration card 300 or test paper, and then the processor 120 recognizes this information by using image recognition technologies. The processor 120 may also obtain the calibration information from the calibration card or test paper in various other ways, which are not further described herein in the present disclosure.

The processor 120 may further be configured to obtain calibration information by remote access. For example, the processor 120 may further be configured to: obtain a unique identifier of the test paper; access an information storage address of the test paper remotely; and obtain the calibration information from the information storage address of the test paper based on the unique identifier. A user may enter the unique identifier of the test paper by using a component such as a keyboard/touch screen and the like on the portable dairy solution analysis device 100B, so that the processor 120 may obtain the unique identifier of the test paper. The processor 120 may also obtain the unique identifier of the test paper by scanning the calibration card 300 or the test paper as described above. Then, the processor 120 may login to the information storage address (e.g., URL) of the test paper via a network interface or transceiver on the portable dairy solution analysis device 100B. The information storage address of the test paper may be built into the memory of the portable dairy solution analysis device 100B or may be obtained by scanning the calibration card 300 or the test paper as described above. Thereafter, the calibration information corresponding to the unique identifier of the test paper is queried by the processor 120 from the information storage address of the test paper based on the unique identifier of the test paper. Similarly, this calibration information includes a production batch of the test paper, an adjustment factor of the test paper, a manufacturer of the test paper, a production time of the test paper, or a production condition of the test paper.

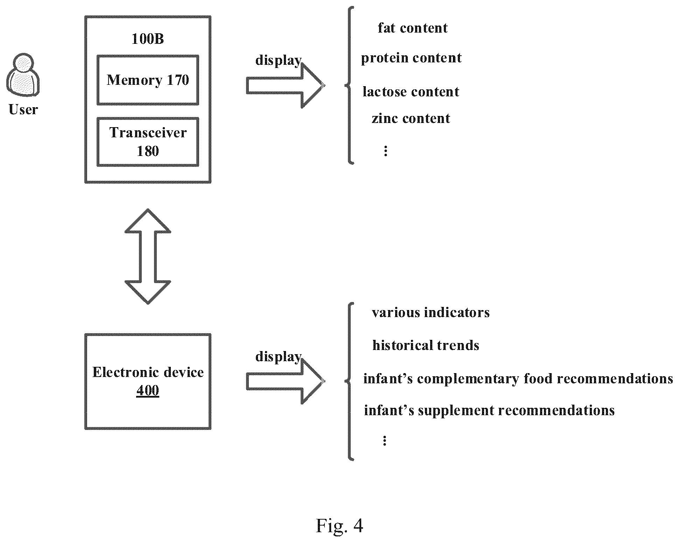

Fig. 4 is another diagram illustrating the portable dairy solution analysis device 100B according to at least one embodiment of the present disclosure, which shows an interaction scenario when the portable dairy solution analysis device 100B is connected to an electronic device 400. The electronic device 400 may be a computing device that includes a processor and a memory, such as a television, a smart appliance device (e.g., a smart refrigerator, a smart microwave oven, etc.), a rechargeable vehicle, a desktop computer, a laptop computer, a smartphone, a tablet computer, a music player (e.g., an mp3 player, etc.), and other terminal including a processor and memory (e.g., a mobile terminal, a smartphone).

The portable dairy solution analysis device 100B further includes a memory 170 and a transceiver 180.

The memory 170 may be configured to store test values of at least one of indicators of the dairy solution. Each time the dairy solution is measured, the memory 170 may store a test value of at least one indicator of the dairy solution obtained in this measurement. In the case where the portable dairy solution analysis device 100B is not connected to the electronic device 400, test values of at least one of the indicators measured for a certain number of times or for a certain period, for example, 50 to 200 groups of test values of the indicators, may be stored by the memory 170.

The transceiver 180 may be configured to send a test value of at least one indicator of the dairy solution stored in the memory 170 to the electronic device 400, in the case that the portable dairy solution analysis device 100B is connected to the electronic device. The portable dairy solution analysis device 100B may be connected to the electronic device 400 via a network. The network may be Internet of Things (IoT) based on Internet or telecommunication network, and may be a wired or wireless network, such as a local area network (LAN), a metropolitan area network (MAN), a wide area network (WAN), a cellular data communication network, and other electronic networks that enable information exchange functions. The portable dairy solution analysis device 100B may also be connected to the electronic device 400 via Bluetooth, infrared transmission, and the like. The specific connection manner of the portable dairy solution analysis device 100B and the electronic device 400 is not limited by the present disclosure.

The transceiver 180 may be configured to transmit and store all test values to the electronic device in sequential order of measurement timing. The electronic device 400 may have software (e.g., a mobile phone application) related to dairy testing stored thereon, which can parse the test values of at least one indicator sent by the dairy device and generate corresponding analysis results. The software may be downloaded by scanning a two-dimensional code or URL on the portable dairy solution analysis device 100B or an instruction manual thereof, or by obtaining a download address over the Internet.

In the case where a test value of at least one indicator of the dairy solution is within a reference range for normal values, the electronic device 400 (or the software thereon) may display an analysis result that the test value of the at least one indicator of the tested dairy solution is normal. In the case where the indicator of the dairy solution deviates from the reference range for normal values, the portable dairy solution analysis device 100B or the electronic device 400 may not only display the analysis result that test valuea of at least one indicator of the tested dairy solution is abnormal, but also perform a detailed analysis for these abnormal indicators. The software on the electronic device 400 may display detailed reports for respective indicators. For example, the electronic device 400 may display a detail page that includes explanations for meanings of various component tests, reference ranges for normal values of various indicators and corresponding analysis results, recommendations and recommended recipe services after synthesizing various results, to enhance the user's sense of use.

In the case where the tested dairy solution is breast milk and values of indicators of the dairy solution deviate from reference ranges for normal values, analysis results of the dairy solution output from the portable dairy solution analysis device 100B or electronic device 400 may further include one or more of: historical trends, mother's health condition, mother's recipe recommendations, infant's complementary food recommendations, infant's supplement recommendations, physician recommendations and breast milk improving courses, and the like.

In the case where the analysis results described above include historical trends, the software on the electronic device 400 may parse the received test values of at least one indicator. Then, in accordance with each indicator, a corresponding historical data trend graph is plotted for the indicator, allowing the user to review previous test results and analysis. The historical data trend graph for each indicator shows most recent 7 test values. When the dairy solution is breast milk, the historical data trend graph may further show reference ranges for normal values of colostrum, transitional milk, and mature milk. Thus, the historical data trend graph may show a changing trend of breast milk and whether it exceeds the reference ranges for normal values. Accordingly, a user may conduct a comparative analysis based on various indicators of breast milk and conditions of infant growth and development.

Optionally, information relating to infants, the elderly, and patients may also be entered into the software on the electronic device 400. For example, a user may enter information relating to an infant's age in month and gender directly into the electronic device 400. The portable dairy solution analysis device 100B may also transmit the information on the infant's age in month and gender recorded therein to the electronic device 400 over a network. With this relevant information, the electronic device 400 may comprehensively analyze the quality of the dairy solution based on various indicators of the dairy solution. Certainly, the portable dairy solution analysis device 100B may also provide similar analysis results of the quality of the dairy solution.

For example, when values of respective indicators of breast milk are within reference ranges for normal values, the portable dairy solution analysis device 100B or electronic device 400 may remind the mother to continue her scientific diet and maintain breastfeeding. When an abnormality is detected in one or more of the indicators of breast milk, the portable dairy solution analysis device 100B or electronic device 400 may remind the mother to consult a professional doctor for analysis based on the clinical diagnosis and an overall situation of the infant, so as to adjust the breastfeeding plan in time to avoid affecting the growth and development of the infant. Abnormalities in one or more of these indicators may also indicate that the mother is suffering from an illness, and the portable dairy analysis device 100B or the electronic device 400 may assess a possibility of the mother's illness and her health status by using these indicators.

The portable dairy solution analysis device 100B or the electronic device 400 may also provide appropriate solutions, for example, recipe recommendations for mothers (e.g., nutritional products, recipes, etc., that can improve breast milk quality may be recommended to mothers) and breast milk improvement courses, to improve the quality of breast milk based on values of various indicators of breast milk. For example, in the case where fat content in breast milk exceeds a reference range for normal values, the portable dairy solution analysis device 100B or the electronic device 400 will remind the mother to maintain a nutritional balance along with light, low-fat foods (e.g., provide corresponding recommended recipes) and provide breast milk improvement course pushes (e.g., exercise videos suitable for breastfeeding mothers, etc.). For another example, if zinc content of breast milk is below a reference range for normal values, the electronic device 400 will remind the mother that she should supplement foods with a high zinc content (e.g., porcine liver, shellfish, etc.) as well as maintain a nutritional balance. The electronic device 400 may also recommend that the mother should supplement appropriate maternal nutrient products, or feed the infant with products containing specific infant supplements and the like. For yet another example, if protein content in breast milk is below a reference range for normal values, the portable dairy solution analysis device 100B or the electronic device 400 may remind the mother that she should supplement foods containing a high amount of high-quality protein (soy products, lean meat, eggs, fish, milk from cows and goats, etc.).

In the case that the dairy solution is cow's milk, goat's milk or formula milk and the indicators of the dairy solution deviate from the reference ranges for normal values, the analysis results of the dairy solution provided by the portable dairy solution analysis device 100B or the electronic device 400 include one or more of: historical trends, infant's complementary food recommendations, infant's supplement recommendations, supplement recipe recommendations, physician recommendations and the like.

Infants may need supplements and complementary foods as they grow older by the month. Complementary foods may be cow's milk, goat's milk, or formula milk. The elderly or sick may also supplement nutrition with cow's milk, goat's milk, or formula milk. For these situations, the portable dairy solution analysis device 100B or the electronic device 400 may further provide corresponding recommendations depending on different people and different types of dairy solutions. For example, the portable dairy solution analysis device 100B or the electronic device 400 may also determine qualities of the cow's milk, goat's milk, or formula milk by testing various indicators of these dairy solutions and provide corresponding historical data trend graphs for users to review previous test results and analysis. Similar to breast milk, the portable dairy solution analysis device 100B or 400 may also provide supplemental recipe recommendations and physician recommendations for infants, the elderly, or patients.

The dairy solution analysis method 100A may further include the following operations: in the case that the test value for the at least one indicator of the dairy solution deviates from a reference range for normal values, it is reminded to reacquire the dairy solution in a first cycle for testing ; and, in the case that the test value for the at least one indicator of the dairy solution is within the reference range for normal values, it is reminded to reacquire the dairy solution in a second cycle for testing , where the second cycle is greater than the first cycle.

Optionally, in the case that the values of the indicators of the dairy solution deviate from the reference ranges for normal values, the portable dairy solution analysis device 100B or the electronic device 400 prompts to reacquire the dairy solution in the first cycle for testing. In the case that the values of the indicators of the dairy solution are within the reference ranges for normal values, the portable dairy solution analysis device 100B or the electronic device 400 prompts to reacquire the dairy solution in the second cycle for testing. The second cycle (e.g., one month) is greater than the first cycle (e.g., 1-2 weeks). The first cycle and the second cycle may also be determined based on at least one of indicators of the infant's month-age dairy solution, thereby adjusting an interval for testing the dairy solution based on different periods of lactation. Furthermore, the user may manually enter the cycle of the dairy solution test.

The portable dairy solution analysis device 100B or the electronic device 400 may output days or hours from the time when a next test is recommended, to remind the user when the next dairy solution test is to be performed. For example, the portable dairy solution analysis device 100B may display or play the information via its display screen or speaker. The portable dairy solution analysis device 100B or the electronic device 400 may also output a recommended date for the next test to remind the user to perform a dairy solution test. Certainly, the portable dairy solution analysis device 100B may also remind the user of the time interval for the dairy solution test in other ways, which is not limited by the present disclosure.

Thus, the interaction between the portable dairy solution analysis device 100B and the electronic device 400 may facilitate the user to learn a situation of the dairy solution, so as to help the user adjust a diet structure according to an actual situation and improve the user experience.

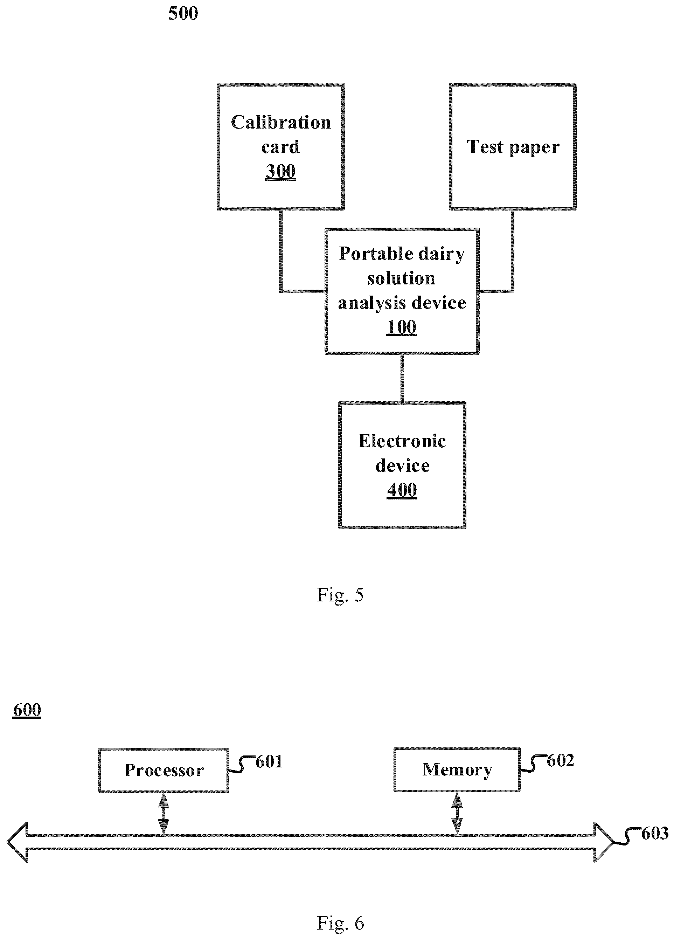

Fig. 5 is a diagram illustrating a dairy solution analysis system 500 according to at least one embodiment of the present disclosure.

The dairy solution analysis system 500 includes the portable dairy solution analysis device 100B and the electronic device 400. The electronic device 400 is configured to provide analysis results of the dairy solution. The portable dairy solution analysis device 100B is configured to calibrate the test value of the at least one indicator of the dairy solution according to the calibration card 300.

The dairy solution analysis system 500 further includes test papers. The portable dairy solution analysis device 100B is configured to detect a test value of at least one indicator of the dairy solution based on a colorimetric biochemical testing method. Values of a plurality of indicators are obtained by using the test papers.

Fig. 6 is a schematic diagram illustrating a dairy solution analysis electronic device 600 used in the present invention.

Referring to Fig. 6, the dairy solution analysis electronic device 600 includes a microprocessor 601 and a memory 602. The microprocessor 601 and the memory 602 may be connected via a bus 603.

The microprocessor 601 may perform various actions and processing based on a program stored in the memory 602. Specifically, the microprocessor 601 may be an integrated circuit chip with signal processing capability. The microprocessor 601 may be a general-purpose processor, a digital signal processor (DSP), an application-specific integrated circuit (ASIC), a field programmable gate array (FPGA) or other programmable logic device, a discrete gate or transistor logic device, or a discrete hardware component. Each of the methods, steps, and logic block diagrams disclosed in the embodiments of this application may be implemented or performed. The general-purpose processor may be a microprocessor or the microprocessor may also be any conventional processor, etc., and maybe of an X86 or ARM architecture.

Computer instructions are stored in the memory 602. The dairy solution analysis method 100A described above is implemented when the computer instructions are executed by the microprocessor 601. The memory 602 may be a volatile memory or a non-volatile memory or may include both a volatile and a non-volatile memory. The non-volatile memory may be a read-only memory (ROM), programmable read-only memory (PROM), erasable programmable read-only memory (EPROM), electrically erasable programmable read-only memory (EEPROM), or flash memory. The volatile memory may be random access memory (RAM), which is used as an external cache. By way of exemplary but not limitative illustration, many forms of RAMs are available, such as a static random access memory (SRAM), a dynamic random access memory (DRAM), a synchronous dynamic random access memory (SDRAM), a double data rate synchronous dynamic random access memory (DDR SDRAM), an enhanced synchronous dynamic random access memory (ES DRAM), a synchronous connecting dynamic random access memory (SLDRAM) and a direct memory bus random access memory (DR RAM). It should be noted that the memory for the methods described herein is intended to include, but not be limited to, these and any other suitable types of memories.

The present disclosure presents a portable dairy solution analysis device, system, method, and computer-readable storage medium. The portable dairy solution analysis device is miniaturized and handheld for ease of operation. A user may complete the entire dairy solution testing process at home. Meanwhile, the present disclosure also discloses software corresponding to this device, and a combination thereof may provide a variety of solutions for the user, including test results, result analysis, suggestions and recommendations for corresponding recipes, products, courses, information, etc., to facilitate the user to learn about the situation of the dairy solution and adjust a diet structure in the light of an actual situation, and provide a healthier infant feeding and patient care methods , thereby improving the user experience.

It should be noted that the flowcharts and block diagrams in the accompanying drawings illustrate possible architectures, functionalities, and operations that may be implemented with the systems, methods, and computer program products in accordance with various embodiments of the present disclosure. In this regard, each box in the flowcharts or block diagrams may represent a module, a segment, or a portion of codes which contains one or more executable instructions for implementing specified logical functions. It should also be noted that in some implementations as alternatives, functions shown in the boxes may also occur in a different order than those shown in the accompanying drawings. For example, two successively illustrated boxes may actually be executed substantially in parallel, and they may also sometimes be executed in a reverse order, depending on functions involved. It should also be noted that each box in the block diagrams and/or flowcharts, and combinations of boxes in the block diagrams and/or flowcharts, may be implemented using a dedicated hardware-based system that performs specified functions or operations, or may be implemented using a combination of dedicated hardware and computer instructions.

In general, various exemplary embodiments of the present disclosure may be implemented in hardware or dedicated circuits, software, firmware, logics, or any combination thereof. Some aspects may be implemented in hardware, while other aspects may be implemented in firmware or software that can be executed by a controller, microprocessor, or other computing devices. When aspects of at least one embodiment of the present disclosure are illustrated or depicted as a block diagram, flowchart, or some other graphical representations, it will be understood that boxes, devices, systems, techniques, or methods described herein may be implemented as non-limiting examples in hardware, software, firmware, dedicated circuits or logics, general-purpose hardware, or controllers or other computing devices, or some combinations thereof.

The accompanying drawings of the embodiments of the present disclosure relate only to the structures involved in the embodiments of the present disclosure, and other structures may refer to common designs. The embodiments of the present disclosure and the features in the embodiments may be combined with each other to obtain new embodiments if there is no conflict.

The exemplary embodiments of the present invention described in detail above are merely illustrative and not limitative. It should be understood by those skilled in the art that various modifications and combinations of these examples or features thereof may be made without departing from the principles of the invention, and such modifications should fall within the scope of the present invention.

The foregoing are merely exemplary embodiments of the present disclosure, and not intended to limit the scope of the disclosure. The scope of the disclosure is determined by the appended claims.

## Claims

1. A dairy solution analysis method (100A), comprising:
controlling a plurality of light emitting diodes to emit light of different wavelengths according to a type of a substance to be tested, wherein different light emitting diodes emit light with different wavelengths;
detecting (101), by internal sensors, a test value of at least one indicator of a dairy solution by emitting lighting on different wavelengths of light on a plurality of test papers (204) , based on a colorimetric biochemical testing method, wherein the detecting (101) including: the internal sensors absorb light reflected by the test papers and determining the test value of the at least one of indicators by a degree of light transmission of the test papers;
obtaining (301) calibration information, wherein the calibration information includes at least one of: a production batch of the test paper, an adjustment factor of the test paper, a manufacturer of the test paper, a production time of the test paper, and a production condition of the test paper;
calibrating (302) the test value of the at least one indicator of the dairy solution according to the calibration information;
outputting (102) an analysis result of the dairy solution based on a calibrated test value of the at least one indicator of the dairy solution,
wherein the plurality of test papers (204) are located in a test paper housing component (200), the test paper housing component (200) is placed on a test paper placement platform (140),
wherein in the case where the calibration information includes the adjustment factor of the test paper, an actual value of the at least one indicator of the dairy solution is obtained by applying the adjustment factor of the test paper to the test value of the at least one indicator of the dairy solution.

2. The dairy solution analysis method of claim 1, wherein,
the analysis result of the dairy solution includes whether the test value of the at least one indicator of the dairy solution deviates from a reference range for normal values.

3. The dairy solution analysis method of claim 2, wherein,
the reference range for normal values is determined based on an infant's age in month and gender.

4. The dairy solution analysis method of claim 1, wherein,
the at least one indicator of the dairy solution includes at least one of: fat content, protein content, lactose content, calories, zinc content, calcium content.

5. The dairy solution analysis method of claim 1, further comprising:
sending the test value of the at least one indicator of the dairy solution to an electronic device (400).

6. The dairy solution analysis method of claim 5, wherein,
an analysis result of the dairy solution is output by the electronic device (400).

7. The dairy solution analysis method of claim 2, wherein,
in a case where the dairy solution is breast milk and the test value of the at least one of indicator of the dairy solution deviates from the reference range for normal values, the analysis result of the dairy solution includes one or more of: historical trends, mother's health condition, mother's recipe recommendations, infant's complementary food recommendations, infant's supplement recommendations, physician recommendations, and breast milk improvement courses.

8. The dairy solution analysis method of claim 2, wherein,
in a case where the dairy solution is cow's milk, goat's milk or formula milk and the test value of the at least one indicator of the dairy solution deviates from the reference range for normal values, the analysis result of the dairy solution includes one or more of: historical trends, infant's complementary food recommendations, infant's supplement recommendations, supplement recipe recommendations and physician recommendations.

9. The dairy solution analysis method of claim 2, wherein,
in a case where the test value of the at least one indicator of the dairy solution deviates from the reference range for normal values, reminding to reacquire the dairy solution in a first cycle for testing; and
in a case where the test value of the at least one indicator of the dairy solution is within the reference range for normal values, reminding to reacquire the dairy solution in a second cycle for testing;
wherein the second cycle is greater than the first cycle.

10. The dairy solution analysis method of claim 9, wherein,
the first cycle and the second cycle are determined based on at least one of an infant's age in month and the test value of the at least one indicator of the dairy solution.

11. A portable dairy solution analysis device (100B), comprising:
a processor (120) configured to control a plurality of light emitting diodes to emit light of different wavelengths according to a type of a substance to be tested, wherein different light emitting diodes emit light with different wavelengths;
a detector (110) configured to detect (101) a test value of at least one indicator of a dairy solution by emitting lighting on different wavelengths of light on a plurality of test papers (204), based on a colorimetric biochemical testing method, wherein the detecting (101) including: the internal sensors absorb light reflected by the test papers and determining the test value of the at least one of indicators by a degree of light transmission of the test papers;
the processor (120) configured to obtain (301) calibration information, wherein the calibration information includes at least one of: a production batch of the test paper, an adjustment factor of the test paper, a manufacturer of the test paper, a production time of the test paper, and a production condition of the test paper and calibrate (302) the test value of the at least one indicator of the dairy solution according to the calibration information;
the processor (120) configured to output an analysis result of the dairy solution based on a calibrated test value of the at least one indicator,
wherein the plurality of test papers (204) are located in a test paper housing component (200), the test paper housing component (200) is placed on a test paper placement platform (140),
wherein in the case where the calibration information includes the adjustment factor of the test paper, an actual value of an indicator is obtained by applying the adjustment factor of the test paper to the test value.

12. A dairy solution analysis system (500), comprising:
the portable dairy solution analysis device (100B) of claim 11,
an electronic device (400) configured to output an analysis result of a dairy solution.

## Patentansprüche

1. Verfahren zur Analyse von Milchlösung (100A), umfassend:
Steuern einer Vielzahl von Leuchtdioden, um Licht unterschiedlicher Wellenlängen in Abhängigkeit von einem Typ einer zu prüfenden Substanz zu emittieren, wobei unterschiedliche Leuchtdioden Licht mit unterschiedlichen Wellenlängen emittieren;
Detektieren (101) mittels interner Sensoren eines Testwertes mindestens eines Indikators einer Milchlösung mittels Aussenden von Licht unterschiedlicher Wellenlängen auf eine Vielzahl von Testpapieren (204), basierend auf einem kolorimetrischen biochemischen Testverfahren, wobei das Detektieren (101) umfasst: die internen Sensoren absorbieren mittels der Testpapiere reflektiertes Licht und Bestimmen des Testwerts des mindestens einen Indikators mittels eines Lichtdurchlässigkeitsgrades der Testpapiere;
Erhalten (301) von Kalibrierungsinformation, wobei die Kalibrierungsinformation mindestens eines von Folgendem umfasst: eine Produktionscharge des Testpapiers, einen Anpassungsfaktor des Testpapiers, einen Hersteller des Testpapiers, einen Produktionszeitpunkt des Testpapiers und eine Produktionsbedingung des Testpapiers;
Kalibrieren (302) des Testwerts des mindestens einen Indikators der Milchlösung gemäß der Kalibrierungsinformation;
Ausgeben (102) eines Analyseergebnisses der Milchlösung basierend auf einem kalibrierten Testwert des mindestens einen Indikators der Milchlösung,
wobei die Vielzahl von Testpapieren (204) in einer Testpapier-Aufnahmekomponente (200) angeordnet sind, die Testpapier-Aufnahmekomponente (200) auf einer Testpapier-Anordnungsplattform (140) angeordnet ist,
wobei in dem Fall, dass die Kalibrierungsinformation den Anpassungsfaktor des Testpapiers umfasst, ein tatsächlicher Wert des mindestens einen Indikators der Milchlösung erhalten wird mittels Anwendung des Anpassungsfaktors des Testpapiers auf den Testwert des mindestens einen Indikators der Milchlösung.

2. Verfahren zur Analyse von Milchlösung gemäß Anspruch 1, wobei
das Analyseergebnis der Milchlösung enthält, ob der Testwert des mindestens einen Indikators der Milchlösung von einem Referenzbereich für Normalwerte abweicht.

3. Verfahren zur Analyse von Milchlösung gemäß Anspruch 2, wobei
der Referenzbereich für Normalwerte basierend auf dem Alter eines Säuglings in Monaten und seines Geschlechts bestimmt wird.

4. Verfahren zur Analyse von Milchlösung gemäß Anspruch 1, wobei
der mindestens eine Indikator der Milchlösung mindestens eines enthält von: Fettgehalt, Proteingehalt, Laktosegehalt, Kalorien, Zinkgehalt, Kalziumgehalt.

5. Verfahren zur Analyse von Milchlösung gemäß Anspruch 1, außerdem umfassend:
Senden des Testwerts des mindestens einen Indikators der Milchlösung an eine elektronische Vorrichtung (400).

6. Verfahren zur Analyse von Milchlösung gemäß Anspruch 5, wobei
ein Analyseergebnis der Milchlösung mittels der elektronischen Vorrichtung (400) ausgegeben wird.

7. Verfahren zur Analyse von Milchlösung gemäß Anspruch 2, wobei
in einem Fall, in dem die Milchlösung Muttermilch ist und der Testwert des mindestens einen Indikators der Milchlösung von dem Referenzbereich für Normalwerte abweicht, das Analyseergebnis der Milchlösung eines oder mehreres enthält von: historische Trends, Gesundheitszustand der Mutter, Rezeptempfehlungen der Mutter, Empfehlungen für Beikost des Säuglings, Präparatempfehlungen des Säuglings, Arztempfehlungen und Muttermilchverbesserungskurse.

8. Verfahren zur Analyse von Milchlösung gemäß Anspruch 2, wobei
in einem Fall, in dem die Milchlösung Kuhmilch, Ziegenmilch oder Säuglingsmilchnahrung ist und der Testwert des mindestens einen Indikators der Milchlösung von dem Referenzbereich für Normalwerte abweicht, das Analyseergebnis der Milchlösung eines oder mehreres enthält von: historische Trends, Empfehlungen für Beikost des Säuglings, Präparatempfehlungen des Säuglings, Ergänzungsrezepturempfehlungen und Arztempfehlungen.

9. Verfahren zur Analyse von Milchlösung gemäß Anspruch 2, wobei
in einem Fall, in dem der Testwert des mindestens einen Indikators der Milchlösung von dem Referenzbereich für Normalwerte abweicht, daran erinnert wird, die Milchlösung in einem ersten Zyklus zum Prüfen erneut zu beschaffen; und
in einem Fall, in dem der Testwert des mindestens einen Indikators der Milchlösung innerhalb des Referenzbereichs für Normalwerte liegt, daran erinnert wird, die Milchlösung in einem zweiten Zyklus zur Prüfung erneut zu beschaffen;
wobei der zweite Zyklus länger als der erste Zyklus ist.

10. Verfahren zur Analyse von Milchlösung gemäß Anspruch 9, wobei
der erste Zyklus und der zweite Zyklus bestimmt werden basierend auf mindestens einem von: einem Alter eines Säuglings in Monaten und dem Testwert des mindestens einen Indikators der Milchlösung.

11. Tragbare Vorrichtung zur Analyse von Milchlösung umfassend:
einen Prozessor (120), der so konfiguriert ist, um eine Vielzahl von Leuchtdioden so zu steuern, dass sie Licht unterschiedlicher Wellenlängen in Abhängigkeit von einem Typ einer zu testenden Substanz emittieren, wobei verschiedene Leuchtdioden Licht mit unterschiedlichen Wellenlängen emittieren;
einen Detektor (110), der konfiguriert ist, um einen Testwert mindestens eines Indikators einer Milchlösung mittels Beleuchtung einer Vielzahl von Testpapieren (204) auf unterschiedlichen Lichtwellenlängen zu detektieren (101), basierend auf einem kolorimetrischen biochemischen Testverfahren, wobei das Detektieren (101) umfasst: die internen Sensoren absorbieren mittels der Testpapiere reflektiertes Licht und Bestimmen des Testwertes des mindestens einen Indikators mittels eines Lichtdurchlässigkeitsgrades der Testpapiere;
wobei der Prozessor (120), konfiguriert ist, um Kalibrierungsinformation zu erhalten (301), wobei die Kalibrierungsinformation mindestens eines enthält von: eine Produktionscharge des Testpapiers, einen Anpassungsfaktor des Testpapiers, einen Hersteller des Testpapiers, einen Produktionszeitpunkt des Testpapiers und eine Produktionsbedingung des Testpapiers, und den Testwert des mindestens einen Indikators der Milchlösung gemäß der Kalibrierungsinformation kalibriert (302);
der Prozessor (120) konfiguriert ist, um ein Analyseergebnis der Milchlösung basierend auf einem kalibrierten Testwert des mindestens einen Indikators auszugeben,
wobei die Vielzahl von Testpapieren (204) in einer Testpapier-Aufnahmekomponente (200) angeordnet ist, die Testpapieraufnahmekomponente (200) auf einer Testpapier-Anordnungsplattform (140) angeordnet ist,
wobei in dem Fall, dass die Kalibrierungsinformation den Anpassungsfaktor des Testpapiers umfasst, ein tatsächlicher Wert eines Indikators mittels Anwenden des Anpassungsfaktors des Testpapiers auf den Testwert erhalten wird.

12. System (500) zur Analyse von Milchlösung, umfassend: die tragbare Vorrichtung (100B) zur Analyse von Milchlösung gemäß Anspruch 11, und einem tragbaren Milchlösungen-Analys, eine elektronische Vorrichtung (400), die konfiguriert ist, um ein Analyseergebnis einer Milchlösung auszugeben.

## Revendications

1. Procédé d'analyse de solution laitière (100A), comprenant :
la commande d'une pluralité de diodes électroluminescentes pour émettre de la lumière de différentes longueurs d'ondes en fonction d'un type d'une substance à tester, dans lequel différentes diodes électroluminescentes émettent de la lumière à différentes longueurs d'ondes ;
la détection (101), par des capteurs internes, d'une valeur de test d'au moins un indicateur d'une solution laitière par émission d'éclairage à différentes longueurs d'ondes de lumière sur une pluralité de bandelettes de test (204), en se basant sur un procédé de test biochimique colorimétrique, dans lequel la détection (101) inclut : les capteurs internes absorbent de la lumière réfléchie par les bandelettes de test et déterminent la valeur de test de l'au moins un indicateur par un degré de transmission de lumière des bandelettes de test ;
l'obtention (301) d'informations de calibrage, dans lequel les informations de calibrage incluent au moins l'une des informations suivantes : un lot de fabrication de la bandelette de test, un facteur d'ajustement de la bandelette de test, un fabricant de la bandelette de test, une heure de fabrication de la bandelette de test et une condition de fabrication de la bandelette de test ;
le calibrage (302) de la valeur de test de l'au moins un indicateur de la solution laitière en fonction des informations de calibrage ;
l'émission (102) d'un résultat d'analyse de la solution laitière en se basant sur une valeur de test calibrée de l'au moins un indicateur de la solution laitière,
dans lequel la pluralité de bandelettes de test (204) se situe dans un composant de boîtier pour bandelettes de test (200), le composant de boîtier pour bandelettes de test (200) étant placé sur une plateforme de placement des bandelettes de test (140),
dans lequel, lorsque les informations de calibrage incluent le facteur d'ajustement de la bandelette de test, une valeur réelle de l'au moins un indicateur de la solution laitière est obtenue en appliquant le facteur d'ajustement de la bandelette de test à la valeur de test de l'au moins un indicateur de la solution laitière.

2. Procédé d'analyse de solution laitière selon la revendication 1, dans lequel le résultat d'analyse de la solution laitière indique si la valeur de test de l'au moins un indicateur de la solution laitière s'écarte d'une plage de référence pour les valeurs normales.

3. Procédé d'analyse de solution laitière selon la revendication 2, dans lequel la plage de référence pour les valeurs normales est déterminée sur la base d'un âge d'un nourrisson en mois et d'un genre.

4. Procédé d'analyse de solution laitière selon la revendication 1, dans lequel l'au moins un indicateur de la solution laitière inclut au moins un de : teneur en graisses, teneur en protéines, teneur en lactose, calories, teneur en zinc, teneur en calcium.

5. Procédé d'analyse de solution laitière selon la revendication 1, comprenant en outre :
l'envoi de la valeur de test de l'au moins un indicateur de la solution laitière à un dispositif électronique (400).

6. Procédé d'analyse de solution laitière selon la revendication 5, dans lequel un résultat d'analyse de la solution laitière est émis par le dispositif électronique (400).

7. Procédé d'analyse de solution laitière selon la revendication 2, dans lequel, lorsque la solution laitière est du lait maternel et la valeur de test de l'au moins un indicateur de la solution laitière s'écarte de la plage de référence pour les valeurs normales, le résultat d'analyse de la solution laitière inclut une ou plusieurs des informations suivantes : historique des tendances, état de santé de la mère, recommandations de recettes pour la mère, recommandations d'aliments complémentaires pour le nourrisson, recommandations de suppléments alimentaires pour le nourrisson, recommandations du médecin et formations sur l'amélioration de la qualité du lait maternel.

8. Procédé d'analyse de solution laitière selon la revendication 2, dans lequel, lorsque la solution laitière est du lait de vache, du lait de chèvre ou du lait maternisé et la valeur de test de l'au moins un indicateur de la solution laitière s'écarte de la plage de référence pour les valeurs normales, le résultat d'analyse de la solution laitière inclut une ou plusieurs des informations suivantes : historique des tendances, recommandations d'aliments complémentaires pour le nourrisson, recommandations de suppléments alimentaires pour le nourrisson, recommandations de recettes de suppléments et recommandations du médecin.

9. Procédé d'analyse de solution laitière selon la revendication 2, dans lequel,
lorsque la valeur de test de l'au moins un indicateur de la solution laitière s'écarte de la plage de référence pour les valeurs normales, rappel de réacquérir la solution laitière dans un premier cycle pour des tests ; et
lorsque la valeur de test de l'au moins un indicateur de la solution laitière se situe dans la plage de référence pour les valeurs normales, rappel de réacquérir la solution laitière dans un second cycle pour des tests ;
dans lequel le second cycle est plus grand que le premier cycle.

10. Procédé d'analyse de solution laitière selon la revendication 9, dans lequel le premier cycle et le second cycle sont déterminés sur la base d'au moins l'un d'un âge de nourrisson en mois et la valeur de test de l'au moins un indicateur de la solution laitière.

11. Dispositif portable d'analyse de solution laitière (100B), comprenant :
un processeur (120) configuré pour commander une pluralité de diodes électroluminescentes afin qu'elles émettent de la lumière de différentes longueurs d'ondes en fonction d'un type d'une substance à tester, dans lequel différentes diodes électroluminescentes émettent de la lumière à différentes longueurs d'ondes ;
un détecteur (110) configuré pour détecter (101) une valeur de test d'au moins un indicateur d'une solution laitière en émettant de l'éclairage à différentes longueurs d'ondes de lumière sur une pluralité de bandelettes de test (204), en se basant sur un procédé de test biochimique colorimétrique, dans lequel la détection (101) inclut : les capteurs internes absorbent de la lumière réfléchie par les bandelettes de test et déterminent la valeur de test de l'au moins un indicateur par un degré de transmission de lumière des bandelettes de test ;
le processeur (120) configuré pour obtenir (301) des informations de calibrage, dans lequel les informations de calibrage incluent au moins l'une des informations suivantes : un lot de fabrication de la bandelette de test, un facteur d'ajustement de la bandelette de test, un fabricant de la bandelette de test, une heure de fabrication de la bandelette de test et une condition de fabrication de la bandelette de test, et calibrer (302) la valeur de test de l'au moins un indicateur de la solution laitière en fonction des informations de calibrage ;
le processeur (120) configuré pour émettre un résultat d'analyse de la solution laitière sur la base d'une valeur de test calibrée de l'au moins un indicateur,
dans lequel la pluralité de bandelettes de test (204) se situe dans un composant de boîtier pour bandelettes de test (200), le composant de boîtier pour bandelettes de test (200) étant placé sur une plateforme de placement des bandelettes de test (140),
dans lequel, lorsque les informations de calibrage incluent le facteur d'ajustement de la bandelette de test, une valeur réelle d'un indicateur est obtenue en appliquant le facteur d'ajustement de la bandelette de test à la valeur de test.

12. Système d'analyse de solution laitière (500), comprenant :
le dispositif portable d'analyse de solution laitière (100B) selon la revendication 11,
un dispositif électronique (400) configuré pour émettre un résultat d'analyse d'une solution laitière.
